# EUROPÄISCHE PATENTSCHRIFT

(11) **EP 0 927 010 B1**
(45) Veröffentlichungstag und Bekanntmachung des Hinweises auf die Patenterteilung: **09.10.2002**
(21) Anmeldenummer: 97942020.5
(22) Anmeldetag: 11.09.1997
(51) Int. Cl.: A61F 2/38

(54) **TIBIA-TEIL EINER KNIEGELENKENDOPROTHESE**
SHINBONE ELEMENT OF KNEE JOINT PROTHESIS
ELEMENT TIBIA D'UNE ENDOPROTHESE DU GENOU

(30) Priorität: 11.09.1996 DE 19636935; 02.10.1996 DE 19640798
(43) Veröffentlichungstag der Anmeldung: 07.07.1999
(73) Patentinhaber: PLUS ENDOPROTHETIK AG, 6343 Rotkreuz (CH)
(72) Erfinder: SCHMOTZER, Hans, CH-5000 Aarau (CH)
(74) Vertreter: Popp, Eugen, Dr.
(86) Internationale Anmeldenummer: EP9704981
(87) Internationale Veröffentlichungsnummer: WO98010721

(56) Entgegenhaltungen:
- EP-A- 0 552 950
- EP-A- 0 636 353
- FR-A- 2 700 263
- US-A- 4 822 362
- US-A- 4 938 769

## Beschreibung

Die Erfindung betrifft einen Tibia-Teil einer Kniegelenkendoprothese mit einer Tibia-Plattform bzw. einem Tibia-Plateau und einem an der distalen Seite der Tibia-Plattform angeordneten Verankerungselement, welches mit der Tibia-Plattform verschraubbar ist.

Beim zementfreien Kniegelenkersatz ist die zusätzliche Stabilisierung einer Tibia-Plattform durch Spongiosa-Schrauben Stand der Technik. Zusätzlich zu den Schrauben sind häufig Zapfen, Stege oder Rippen, die fest mit dem Plateau verbunden sind, üblich. Der Vorteil von Schrauben liegt darin, daß aufgrund der dadurch bewirkten Vorspannung eine Kompressionskraft zwischen der Tibia-Plattform und dem Knochen während der Implantation erzeugt wird. Dadurch wird die Primärstabilität erhöht. Des weiteren wird das Einwachsen von Knochen in die distale bzw. Rückfläche der Tibia-Plattform gefördert. Die Verankerung mit Schrauben hat jedoch den Nachteil, daß diese zurückwandern können, wenn es zu Osteonekrosen bzw. Osteolysen unter der Tibia-Plattform kommt. Des weiteren besteht bei der vorgenannten Konstruktion die Gefahr, daß Polyethylen-Abriebspartikel, die von dem auf der proximalen Seite der Tibia-Plattform montierten Polyethylen-Inlay stammen, durch die in der Tibia-Plattform ausgebildeten Schraubenlöcher hindurch in den Knochen wandern. Dies führt dann zu lokalen Osteolysen.

In der einschlägigen Literatur sind auch verschiedene zylindrische Spreizdübel an der distalen Seite der Tibia-Plattform beschrieben. Diese haben den Vorteil, daß sie eine Vorspannkraft ermöglichen. Da sie jedoch auf dem Spreizprinzip beruhen, ist nicht sichergestellt, daß der Dübel fest mit der Tibia-Plattform verbunden bleibt. Es besteht die Möglichkeit eines Zurückwanderns und damit der Ausbildung eines Spalts zwischen Spreizdübel und Tibia-Plateau. Durch diesen Spalt können Polyethylen-Partikel, die vom erwähnten Inlay stammen, in den Knochen wandern und zu Osteolysen führen. Zusätzlich ist die punktuelle Abstützung, die durch Dübel erzeugt wird, nachteilig für eine langfristige Osteointegration des Implantats.

Ein Verankerungselement das durch eine vertikale Wand gekennzeichnet ist, ist aus FR-A-2 700 263 bekannt.

Eine Alternative zu kurzen Dübeln ist die Verwendung von einem langen intramedullären Stiel. Dieser Stiel kann durch Rippen ergänzt sein, wodurch eine etwas breitflächigere Abstützung der Tibia-Plattform erhalten wird. Eine derartige Konstruktion ist z. B. bekannt aus der US-A 4 938 769. Die Verwendung eines intramedullären Stiels hat jedoch den Nachteil, daß die Lastübertragung im distalen Bereich stattfindet. Der proximale Knochen unterhalb der Tibia-Plattform wird im Gegensatz dazu nur gering belastet. Dieser Effekt, das sogenannte stress shielding führt zur Knochenresorption und langfristig zur Lockerung des Implantats.

Es wäre deshalb von Vorteil, wenn durch eine geeignete Konstruktion einerseits eine Vorspannung während der Implantation erzeugt werden könnte und gleichzeitig die Tibia-Plattform und die Verankerungselemente eine Einheit bilden würden, so daß erstens ein Zurückwandern der Verankerungselemente unmöglich wird und zweitens eine hermetische Abdichtung gegenüber Polyethylen-Abriebspartikel entsteht.

Dieses Ziel wird durch einen Tibia-Teil gemäß dem kennzeichnenden Teil des Anspruches 1 erreicht. Dementsprechend ist erfindungsgemäß das Verankerungselement durch ein in Stirnansicht U-, C- oder V-förmig gestaltetes Schild gebildet, dessen Höhe kleiner ist als Breite. Entscheidend ist also, daß das Verankerungsschild im implantierten Zustand eine größere horizontale Erstreckung aufweist als vertikale Erstreckung bzw. Höhe. Durch die relativ geringe Höhe des Verankerungsschildes wird verhindert, daß die Lastübertragung distal erfolgt. Gleichzeitig bietet die großflächige Abstützung im Knochen einen erhöhten Widerstand gegen horizontale Schubkräfte und damit ein Abkippen der Tibia-Plattform. Um während der Implantation eine Vorspannkraft zu erzeugen, wird das Verankerungsschild zunächst im proximalen Tibiaknochen eingeschlagen, und zwar soweit, daß die obere Kante des Verankerungsschildes um einen kleinen Betrag unterhalb der Knochenoberfläche versenkt ist. Anschließend wird die Tibia-Plattform auf das im Knochen versenkte Verankerungsschild geschraubt. Dadurch wird eine Vorspannkraft zwischen Tibia-Plattform und Knochen aufgrund der Reibung zwischen Verankerungsschild und Knochen erzeugt. Da keine Spreizwirkung vorliegt, kann das Schild nach proximal wandern und somit bündig und spielfrei mit der Tibia-Plattform verschraubt werden, ohne daß die Vorspannkraft nennenswert verlorengeht.

Vorzugsweise ist die distale Kante des Verankerungsschildes bogenförmig, insbesondere konvexbogenförmig gestaltet, wobei die Bogenlinie einen Kreisbogen-, Ellipsenabschnitt oder eine Parabel definiert. Durch diese Konfiguration läßt sich das Verankerungsschild leichter in den proximalen Tibiaknochen einschlagen. Im übrigen sei darauf hingewiesen, daß zur Vermeidung eines Verlustes der Vorspannkraft die Wandflächen des Verankerungsschildes sich parallel zueinander erstrecken. Dies gilt auch für die Ausführungsform gemäß Anspruch 10, bei dem die Dicke bzw. Wandstärke des Verankerungsschildes von proximal nach distal abgestuft abnimmt.

Um eine noch bessere Verankerung im proximalen Tibiaknochen zu erreichen, umfaßt das Verankerungsschild gemäß einer weiteren Ausführungsform im Bereich maximaler Höhe bzw. auf etwa halber Breite einen Mittelsteg, der sich nach Art einer winkelhalbierenden zwischen dem lateralen und medialen Abschnitt des Verankerungsschildes erstreckt. Bei Bedarf kann diametral dazu ein weiterer Verankerungssteg angeordnet sein. Es hat sich jedoch gezeigt, daß in der Regel der vorgenannte Mittelsteg für schwierige Verankerungsfälle ausreicht. Dieser Mittelsteg erstreckt sich vorzugsweise über die gesamte Höhe des Verankerungsschildes. Es ist jedoch auch denkbar, den Mittelsteg etwas kürzer als die entsprechende Höhe des Verankerungsschildes auszubilden.

Zur Befestigung des Verankerungsschildes an der distalen Seite der Tibia-Plattform weist das Verankerungsschild wenigstens zwei, insbesondere drei von proximal her zugängliche Gewindebohrungen auf, in die sich durch korrespondierende Bohrungen in der Tibia-Plattform hindurcherstreckende Schrauben zur Befestigung der Tibia-Plattform am Verankerungsschild einschraubbar sind. Die Gewindebohrungen sind vorzugsweise zentral und am lateralen sowie medialen Ende des Verankerungsschildes angeordnet. Zu diesem Zweck ist das Verankerungsschild an den zugeordneten Stellen jeweils zapfenartig ausgebildet.

Zum erleichterten Einschlagen des Verankerungsschildes in den Tibiaknochen kann die distale Kante des Verankerungsschildes als Schneide ausgebildet sein.

Eine weitere Ausführungsform zeichnet sich dadurch aus, daß an der distalen Seite der Tibia-Plattform eine nutenförmige Vertiefung eingearbeitet ist, in die das Verankerungsschild mit seiner proximalen Stirnseite spielfrei einpaßbar ist.

Zur Erhöhung der Vorspannkraft des Verankerungsschildes im Tibiaknochen ist die Oberfläche desselben vorzugsweise aufgerauht, insbesondere strukturiert, und zwar längsstrukturiert. Dadurch geht die Vorspannkraft auch dann nicht verloren, wenn das Verankerungsschild beim Festschrauben der Tibia-Plattform nach proximal wandert. Entscheidend ist die Vergrößerung der Kontaktfläche zwischen Verankerungsschild und Tibiaknochen.

Nachstehend werden bevorzugte Ausführungsformen eines erfindungsgemäß ausgebildeten Tibia-Teils anhand der beigefügten Zeichnungen näher erläutert. Es zeigen:
- Figur 1: eine erste Ausführungsform eines erfindungsgemäßen Tibia-Teils in perspektivischer Explosionsdarstellung;
- Figur 2: eine zweite Ausführungsform eines erfindungsgemäß ausgebildeten Tibia-Teils in perspektivischer Explosionsdarstellung;
- Figur 3: eine weitere Ausführungsform eines erfindungsgemäß ausgebildeten Tibia-Teils in perspektivischer Explosionsdarstellung.

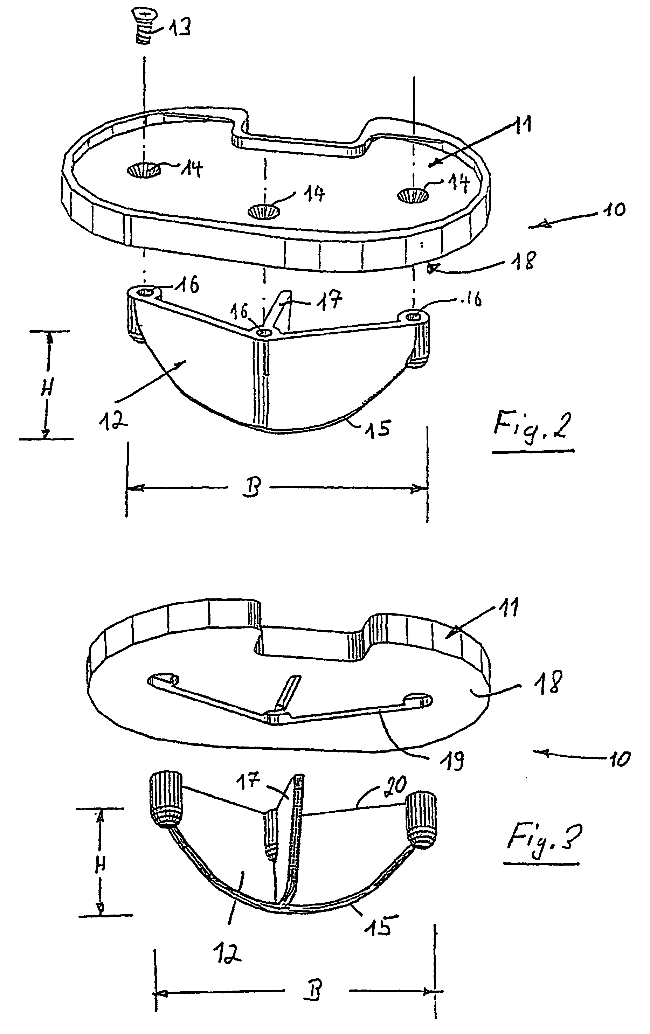

Das in Figur 1 dargestellte Tibia-Teil ist mit der übergeordneten Bezugsziffer 10 gekennzeichnet. Es umfaßt eine Tibia-Plattform bzw. ein Tibia-Plateau 11 und ein an der distalen Seite angeordnetes Verankerungselement in Form eines Verankerungsschildes 12. Das Verankerungsschild ist in Stirnansicht V-förmig abgewinkelt. Die Höhe "H" ist merklich kleiner als die Breite "B". Bei der dargestellten Ausführungsform beträgt die Höhe "H" des Verankerungsschildes 12 etwa die Hälfte der projizierten Breite "B". Statt der V-förmigen Abwinkelung kann das Verankerungsschild 12 in Stirnansicht auch U- oder C-förmig gestaltet sein.

Das Verankerungsschild 12 wird mittels Schrauben 13, die sich durch Bohrungen 14 in der Tibia-Plattform 11 hindurcherstrecken mit dieser verschraubt derart, daß die proximale Stirnseite des Verankerungsschildes vollflächig an der distalen Seite der Tibia-Plattform anliegt.

Die distale Kante 15 des Verankerungsschildes 12 ist bogenförmig, nämlich konvexbogenförmig gestaltet. Die Bogenlinie kann einen Kreisbogenabschnitt, Ellipsenabschnitt oder eine Parabel definieren.

Zum Zwecke der Verschraubung von Verankerungsschild 12 und Tibia-Plattform 11 weist das Verankerungsschild 12 drei von proximal her zugängliche Gewindebohrungen 16 auf, in die sich die durch korrespondierende Bohrungen 14 in der Tibia-Plattform 12 hindurcherstreckenden Schrauben 13 zur Befestigung der Tibia-Plattform 11 am Verankerungsschild 12 einschraubbar sind. Die Schrauben 13 sind Linsen- oder Flachkopfschrauben.

Die Gewindebohrungen 16 sind bei dem dargestellten Ausführungsbeispiel zentral, d. h. im Bereich der Abwinkelung des Verankerungsschildes und am lateralen sowie medialen Ende desselben angeordnet bzw. ausgebildet. Zu diesem Zweck ist das Verankerungsschild an diesen Stellen etwa zapfenartig verdickt ausgebildet. Die distale Kante 15 des Verankerungsschildes 12 kann vorzugsweise noch als Schneide ausgebildet sein.

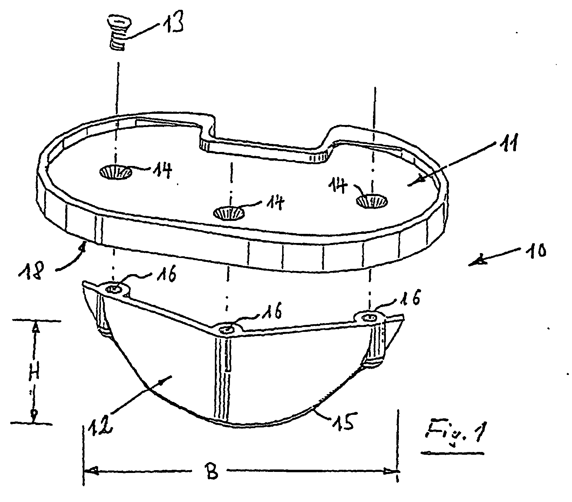

Die Oberfläche des Verankerungsschildes ist aufgerauht, insbesondere längsstrukturiert. Bei der Ausführungsform gemäß Figur 3, die weiter unten noch näher beschrieben wird, kann die Oberflächenstrukturierung auch quer verlaufen, da bei dieser Ausführungsform das Verankerungsschild nach dem Einschlagen in den Tibiaknochen unverrückbar bleibt. Das Verankerungsschild wandert auch beim Befestigen der Tibia-Plattform nicht nach proximal aus.

Die Ausführungsform nach Figur 2 unterscheidet sich von derjenigen nach Figur 1 dadurch, daß im Bereich maximaler Höhe bzw. auf etwa halber Breite das Verankerungsschild einen Mittelsteg 17 umfaßt, der sich nach Art einer Winkelhalbierenden zwischen dem lateralen und medialen Abschnitt des Verankerungsschildes 12 erstreckt. Im übrigen entspricht diese Ausführungsform derjenigen nach Figur 1, so daß sich eine weitere Beschreibung derselben erübrigt. Der Mittelsteg 17 ist also zentral angeordnet und im implantierten Zustand nach dorsal gerichtet. Er bietet eine zusätzliche Abstützung für die Tibia-Plattform. Es wird das posteriore Durchschwingen der Tibia-Plattform reduziert, wenn die resultierende Gelenkskraft nach dorsal wandert, wie es in der Beugung der Fall ist. Des weiteren sei zu der Ausführungsform nach Figur 2 noch erwähnt, daß dort die zapfenartigen Verdickungen zur Ausbildung der Gewindebohrungen 16 den lateralen und medialen Abschluß des Verankerungsschildes 12 bilden. Der zentrale bzw. Mittelsteg 17 kann ebenfalls endseitig mit einer solchen zapfenartigen Verdickung zur Ausbildung einer Gewindebohrung versehen sein.

Der Mittelsteg 17 erstreckt sich vorzugsweise über die gesamte Höhe "H" des Verankerungsschildes 12, so wie dies die Ausführungsform nach Figur 3 erkennen läßt. Es ist jedoch auch denkbar, die Höhe des Mittelsteges 17 etwas reduziert auszubilden.

Die Ausführungsform nach Figur 3 unterscheidet sich von der Ausführungsform nach den Figuren 1 und 2 dadurch, daß an der distalen Seite 18 der Tibia-Plattform 11 eine nutenförmige Vertiefung 19 eingearbeitet ist, in die das Verankerungsschild 12 mit seiner proximalen Stirnseite 20 spielfrei einpaßbar ist. Die Geometrie der nutenartigen Vertiefung 19 entspricht natürlich der Geometrie der Stirnseite 20 des Verankerungsschildes 12. Diese Ausführungsform besitzt den Vorteil, daß das Verankerungsschild nicht vertieft in den Tibiaknochen eingeschlagen werden muß, da der für die Verspannung notwendige Spalt bereits in die Tibia-Plattform eingearbeitet ist. Es besteht der weitere Vorteil, daß die Abdichtung zwischen Tibia-Plattform und Verankerungsschild verbessert wird.

Die Außenkannte des Mittelsteges 17 ist in Richtung von proximal nach distal zum Verankerungsschild 12 hin gerichtet. Dadurch wird das Einschlagen des Verankerungsschildes 12 in den Tibiaknochen erleichtert.

Die proximale Seite der Tibia-Plattform 11 ist in herkömmlicher Weise zur Aufnahme eines Polyethylen-Inlays ausgebildet.

Der beschriebene Tibia-Teil besteht aus einem biokompatiblem Material, insbesondere Titan oder Titanlegierung, wie sie für die Endoprothetik üblich ist.

Die zweiteilige Ausbildung des beschriebenen Tibia-Teils ist herstellungstechnisch vergleichsweise einfach. So wird zum Beispiel in der DE 30 13 155 C2 eine einteilige Ausführungsform dargestellt und beschrieben. Diese ist herstellungstechnisch äußerst aufwendig. Es werden komplizierte Formen benötigt. Auch ist die Nachbearbeitung nur mit großem Aufwand möglich. Des weiteren weist diese bekannte Ausführungsform die oben anhand der US-A 4 938 769 geschilderten Nachteile des sogenannten stress shieldings auf.

Es sei noch darauf hingewiesen, daß die distale Kante 15 bei der Ausführungsform nach den Figuren 2 und 3 zwischen Mittelsteg 17 und lateralem bzw. medialem Ende des Verankerungsschildes 12 jeweils geradlinig oder konkav gebogen verlaufen kann.

Es sei noch erwähnt, daß die distale bzw. Unterseite 18 der Tibia-Plattform 11 vorzugsweise mit im Abstand voneinander angeordneten Vorsprüngen versehen ist. Die Vorsprünge können die Form von Rippen, Schneiden, Zapfen, Kegel, Nadeln oder dergleichen besitzen. Durch derartige Vorsprünge wird ein Anwachsen des Knochens an die Plattform 11 begünstigt.

Versuche haben gezeigt, daß ein gegenseitiger Abstand der erwähnten Vorsprünge in der Größenordnung von 3 bis 6 mm, insbesondere etwa 4 bis 5 mm, besonders vorteilhaft ist für das Anwachsen des Knochens. Die Höhe der Vorsprünge beträgt etwa 0,5 bis 2,5 mm, insbesondere etwa 1,0 mm.

Schließlich sei noch erwähnt, daß es vorteilhaft ist, die Oberfläche des Verankerungsschildes mit einer Querstruktur zu versehen, insbesondere in Form von Querrippen oder Querzähnen. Dadurch läßt sich der Halt des Verankerungsschildes 12 im Knochen erhöhen, ohne daß die Verbindung des Schildes mit der Tibia-Plattform behindert wird.

Die erwähnte Quer-Strukturierung führt zu einer Art Verhakung des Verankerungsschildes im Knochen. Sofern eine Längsstruktur vorgesehen ist, wird dadurch ebenfalls die Verankerung im Knochen erhöht, und zwar aufgrund der vergrößerten Kontaktfläche zwischen Verankerungsschild und Knochen.

Sämtliche in den Anmeldungsunterlagen offenbarten Merkmale werden als erfindungswesentlich beansprucht, soweit sie einzeln oder in Kombination gegenüber dem Stand der Technik neu sind.

### Bezugszeichenliste:

- 10: Tibia-Teil
- 11: Tibia-Plattform
- 12: Verankerungsschild
- 13: Schrauben
- 14: Bohrung
- 15: distale Kante
- 16: Gewindebohrung
- 17: Mittelsteg
- 18: distale Seite
- 19: nutenförmige Vertiefung
- 20: proximale Stirnseite

## Patentansprüche

1. Tibia-Teil einer Kniegelenkendoprothese mit einer Tibia-Plattform (Tibia-Plateau 11) und einem an der distalen Seite derselben angeordneten Verankerungselement (12), welches mit der Tibia-Plattform (11) verschraubbar ist,
**dadurch gekennzeichnet, daß**
das Verankerungselement durch ein in Stirnansicht U-, C-oder V-förmig gestaltetes Schild (12) gebildet ist, dessen Höhe (H) kleiner ist als Breite (B).

2. Tibia-Teil nach Anspruch 1,
**dadurch gekennzeichnet, daß**
die distale Kante (15) des Verankerungsschildes (12) bogenförmig, insbesondere konvexbogenförmig gestaltet ist, wobei die Bogenlinie einen Kreisbogen-, Ellipsenabschnitt, eine Parabel oder dergleichen definiert.

3. Tibia-Teil nach Anspruch 1 oder 2,
**dadurch gekennzeichnet, daß**
das Verankerungsschild (12) im Bereich maximaler Höhe (H) bzw. auf etwa halber Breite (B) einen Mittelsteg (17) umfaßt, der sich nach Art einer Winkelhalbierenden zwischen dem lateralen und medialen Abschnitt des Verankerungsschildes (12) erstreckt.

4. Tibia-Teil nach Anspruch 3,
**dadurch gekennzeichnet, daß** der Mittelsteg (17) sich über die gesamte Höhe (H) des Verankerungsschildes (12) erstreckt.

5. Tibia-Teil nach einem der Ansprüche 1 bis 4,
**dadurch gekennzeichnet, daß**
das Verankerungsschild (12) wenigstens 2, insbesondere 3 von proximal her zugängliche Gewindebohrungen (16) aufweist, in die sich durch korrespondierende Bohrungen (14) in der Tibia-Plattform (11) hindurcherstreckende Schrauben (13) zur Befestigung der Tibia-Plattform (11) am Verankerungsschild (12) einschraubbar sind.

6. Tibia-Teil nach Anspruch 5,
**dadurch gekennzeichnet, daß**
die Gewindebohrungen (16) zentral und am lateralen sowie medialen Ende des Verankerungsschildes (12) angeordnet bzw. ausgebildet sind.

7. Tibia-Teil nach einem der Ansprüche 1 bis 6,
**dadurch gekennzeichnet, daß**
die distale Kante (15) des Verankerungsschildes (12) als Schneide ausgebildet ist.

8. Tibia-Teil nach einem der Ansprüche 1 bis 7,
**dadurch gekennzeichnet, daß**
das Verankerungsschild (12) proximal spielfrei und vollflächig an der distalen Seite (18) der Tibia-Plattform (11) anschließbar ist.

9. Tibia-Teil nach einem der Ansprüche 1 bis 8,
**dadurch gekennzeichnet, daß**
an der distalen Seite (18) der Tibia-Plattform (11) eine nutenförmige Vertiefung (19) eingearbeitet ist, in die das Verankerungsschild (12) mit seiner proximalen Stirnseite (20) spielfrei einpaßbar ist.

10. Tibia-Teil nach einem der Ansprüche 1 bis 9,
**dadurch gekennzeichnet, daß**
die Dicke bzw. Wandstärke des Verankerungsschildes (12) von proximal nach distal abgestuft abnimmt, wobei die Wandflächen der jeweils relativ zueinander abgestuften Abschnitte sich parallel zueinander erstrecken.

11. Tibia-Teil nach einem der Ansprüche 1 bis 10,
**dadurch gekennzeichnet, daß**
die Oberfläche des Verankerungsschildes (12) aufgerauht, insbesondere strukturiert ist

12. Tibia-Teil nach einem der Ansprüche 1 bis 11,
**dadurch gekennzeichnet, daß**
die distale bzw. Unterseite (18) der Tibia-Plattform (11) mit im Abstand voneinander angeordneten Vorsprüngen, insbesondere in Form von Rippen, Schneiden, Zapfen, Nadeln, Kegel oder dergleichen versehen ist, die ein Anwachsen des Knochens an die Plattform (11) begünstigen.

13. Tibia-Teil nach Anspruch 12,
**dadurch gekennzeichnet, daß**
die Vorsprünge an der distalen Seite (18) der Tibia-Plattform (11) einen Abstand von etwa 3 - 6 mm, insbesondere etwa 4 - 5 mm, voneinander aufweisen.

14. Tibia-Teil nach einem der Ansprüche 11 - 13,
**dadurch gekennzeichnet, daß**
die Oberfläche des Verankerungsschildes (12) längs und/oder quer strukturiert, insbesondere mit Längsund/oder Querrippen, -zähnen oder dergleichen versehen ist.

## Claims

1. A tibia part of a stifle joint endoprosthesis, including a tibia platform (tibia plateau 11), and an anchoring element (12) arranged on the distal side of same, which is screw-connectable with the tibia platform (11),
**characterized in that**
said anchoring element is formed by a shield (12) configured U-shaped, C-shaped or V-shaped in the front view, the height (H) of which is smaller than the width (B).

2. The tibia part of claim 1,
**characterized in that**
the distal edge (15) of the anchoring shield (12) is configured bow-shaped, in particular convexly bow-shaped, the bow line defining an arc of a circle portion, an ellipse portion, a parabola or such like.

3. The tibia part of claim 1 or 2,
**characterized in that**
the anchoring shield (12) comprises in the zone of maximum height (H) or approximately at half-width (B), a central web (17) extending in the manner of a median line between the lateral and medial portion of the anchoring shield.

4. The tibia part of claim 3,
**characterized in that**
said central web (17) extends over the entire height (H) of the anchoring shield (12).

5. The tibia part of any one of claims 1 through 4,
**characterized in that**
said anchoring shield (12) has at least 2, in particular 3, proximally accessible thread bores (16), into which screws (13) for fixing the tibia platform (11) on the anchoring shield (12) extending through corresponding bores (14) in the tibia platform (11), can be screwed.

6. The tibia part of claim 5,
**characterized in that**
said thread bores (16) are arranged and/or configured centrally and at the lateral, as well as the medial end of the anchoring shield (12).

7. The tibia part of any one of claims 1 through 6,
**characterized in that**
the distal edge (15) of the anchoring shield (12) is configured as a cutting edge.

8. The tibia part of any one of claims 1 through 7,
**characterized in that**
said anchoring shield (12) is connectable proximally free from play, and all-over its surface to the distal side (18) of the tibia platform (11).

9. The tibia part of any one of claims 1 through 8,
**characterized in that**
at the distal side (18) of the tibia platform (11), a groove-shaped indentation (19) is incorporated, into which the anchoring shield (12) is adaptable free from play with its proximal front side (20).

10. The tibia part of any one of claims 1 through 9,
**characterized in that**
the thickness or wall thickness of the anchoring shield (12) decreases in steps from the proximal to the distal direction, the wall faces of the portions each stepped relative to each other extending in parallel to each other.

11. The tibia part of any one of claims 1 through 10,
**characterized in that**
the surface of the anchoring shield (12) is roughened, in particular textured.

12. The tibia part of any one of claims 1 through 11,
**characterized in that**
the distal or lower side (18) of the tibia platform (11) is provided with protrusions arranged spaced apart from each other, in particular in the form of ribs, cutting edges, spigots, pins, cones or similar, that favor the bone growing on the platform (11).

13. The tibia part of claim 12,
**characterized in that**
the protrusions on the distal side (18) of the tibia platform (11) have a spacing of about 3 - 6 mm, in particular about 4 - 5 mm from each other.

14. The tibia part of any one of claims 11 through 13,
**characterized in that**
the surface of the anchoring shield (12) is textured longitudinally and/or transversely, in particular is provided with longitudinal and/or transverse ribs, longitudinal and/or transverse teeth or similar.

## Revendications

1. Partie tibia d'une endoprothèse d'articulation de genou avec une plate-forme de tibia (plateau de tibia 11) et un élément d'ancrage (12) disposé au côté distal de celle-ci, vissable avec la plate-forme de tibia (11),
**caractérisée en ce que**
l'élément d'ancrage est formé par une plaque (12) de vue frontale en forme de U, de C ou de V, dont la hauteur (H) est plus petite que la largeur (B).

2. Partie tibia selon la revendication 1,
**caractérisée en ce que**
l'arête distale (15) de la plaque d'ancrage (12) se présente en forme d'arc, en particulier d'arc de forme convexe, la ligne d'arc définissant une section d'arc circulaire, elliptique, une parabole ou semblable.

3. Partie tibia selon la revendication 1 ou 2,
**caractérisée en ce que**
la plaque d'ancrage (12) dans la zone de la hauteur maximale (H) ou à mi-largeur (B), comprend une entretoise centrale (17) qui s'étend comme une bissectrice entre la section latérale et médiale de la plaque d'ancrage (12).

4. Partie tibia selon la revendication 3,
**caractérisée en ce que**
l'entretoise centrale (17) s'étend sur la hauteur totale (H) de la plaque d'ancrage (12).

5. Partie tibia selon l'une des revendications 1 à 4,
**caractérisée en ce que**
la plaque d'ancrage (12) présente au moins 2, en particulier 3 perçages filetés (16) accessibles depuis la proximité dans lesquels par des perçages correspondants (14) traversant la plate-forme de tibia (11), des vis (13) de fixation de la plate-forme de tibia (11) à la plaque d'ancrage (12) peuvent se visser.

6. Partie tibia selon la revendication 5,
**caractérisée en ce que**
les perçages filetés (16) sont disposés ou formés de manière centrale ainsi qu'à l'extrémité latérale et médiale de la plaque d'ancrage (12).

7. Partie tibia selon l'une des revendications 1 à 6,
**caractérisée en ce que**
l'arête distale (15) de la plaque d'ancrage (12) est formée en tant que fil tranchant.

8. Partie tibia selon l'une des revendications 1 à 7,
**caractérisée en ce que**
la plaque d'ancrage (12) est raccordable de manière proximale sans jeu et sur la surface totale au côté distal (18) de la plate-forme de tibia (11).

9. Partie tibia selon l'une des revendications 1 à 8,
**caractérisée en ce**
**qu'**au côté distal (18) de la plate-forme de tibia (11), un évidement en forme de gorge (19) a été usiné, dans lequel la plaque d'ancrage (12) peut être insérée sans jeu par sa face frontale proximale (20).

10. Partie tibia selon l'une des revendications 1 à 9,
**caractérisée en ce que**
l'épaisseur de paroi de la plaque d'ancrage (12) décroît par degrés du proximal vers le distal, les surfaces de paroi des sections dégradées relativement les unes aux autres s'étendant parallèlement entre elles.

11. Partie tibia selon l'une des revendications 1 à 10,
**caractérisée en ce que**
la surface de la plaque d'ancrage (12) est rendue rugueuse, en particulier structurée.

12. Partie tibia selon l'une des revendications 1 à 11,
**caractérisée en ce que**
la face distale ou inférieure (18) de la plate-forme de tibia (11) est pourvue de saillies espacées les unes des autres, en particulier en forme de nervures, de fils tranchants, de tourillons, d'aiguilles, de cônes ou similaires, qui favorisent la croissance de l'os contre la plate-forme (11).

13. Partie tibia selon la revendication 12,
**caractérisée en ce que**
les saillies du côté distal (18) de la plate-forme de tibia (11) présentent entre elles un écartement de 3 mm à 6 mm, en particulier de 4 mm à 5 mm.

14. Partie tibia selon l'une des revendications 11 - 13,
**caractérisée en ce que**
la surface de la plaque d'ancrage (12) est pourvue de dents ou similaires, en particulier de nervures en longueur et/ou en travers, structurées en longueur et/ou en travers.
